# EUROPEAN PATENT SPECIFICATION

(11) **EP 2 533 803 B1**
(45) Date of publication and mention of the grant of the patent: **20.01.2016**
(21) Application number: 11702667.4
(22) Date of filing: 11.02.2011
(51) Int. Cl.: A61K 38/48, A61K 35/747, A23L 1/03, A61P 29/00

(54) **LACTOCEPINS FOR USE IN THE TREATMENT OF IP-10-MEDIATED INFLAMMATORY DISEASES**
LACTOCEPINE ZUR BEHANDLUNG VON IP-10-VERMITTELTEN ENTZÜNDUNGSERKRANKUNGEN
LACTOCÉPINES INTERVENANT DANS LE TRAITEMENT DE MALADIES INFLAMMATOIRES INDUITES PAR IP-10

(30) Priority: 11.02.2010 EP 10001419
(43) Date of publication of application: 19.12.2012
(73) Proprietor: Technische Universität München, 80333 München (DE)
(72) Inventor: HALLER, Dirk, 85354 Freising (DE); HÖRMANNSPERGER, Gabriele, 84155 Bodenkirchen (DE)
(74) Representative: Weickmann & Weickmann
(86) International application number: PCT/EP2011/052047
(87) International publication number: WO 2011/098568

(56) References cited:
- OTTE JAN-MICHEL ET AL: "Probiotics regulate the expression of COX-2 in intestinal epithelial cells.", NUTRITION AND CANCER 2009 LNKD- PUBMED:19116880, vol. 61, no. 1, 2009, pages 103-113, XP009147285, ISSN: 1532-7914
- ANTONIO TURSI ET AL: "Probiotics regulate the expression of COX-2 in intestinal epithelial cells.", INTERNATIONAL JOURNAL OF COLORECTAL DISEASE ; CLINICAL AND MOLECULAR GASTROENTEROLOGY AND SURGERY, SPRINGER, BERLIN, DE, vol. 22, no. 9, 28 March 2007 (2007-03-28) , pages 1103-1108, XP019541291, ISSN: 1432-1262, DOI: DOI:10.1007/S00384-007-0299-6
- HOERMANNSPERGER GABRIELE ET AL: "Post-translational inhibition of IP-10 secretion in IEC by probiotic bacteria: impact on chronic inflammation", PLOS ONE, PUBLIC LIBRARY OF SCIENCE, SAN FRANCISCO, CA, US, vol. 4, no. 2, 1 January 2009 (2009-01-01) , pages E4365-1, XP009147278, ISSN: 1932-6203
- HOLCK A ET AL: "CLONING, SEQUENCING AND EXPRESSION OF THE GENE ENCODING THE CELL-ENVELOPE-ASSOCIATED PROTEINASE FROM LACTOBACILLUS PARACASEI SUBSP. PARACASEI NCDO 151", JOURNAL OF GENERAL MICROBIOLOGY, SOCIETY FOR MICROBIOLOGY, READING, GB, vol. 138, no. PART 07, 1 July 1992 (1992-07-01), pages 1353-1364, XP000608160, ISSN: 0022-1287 cited in the application
- HÖRMANNSPERGER GABRIELE ET AL: "Posttranslational inhibition of proinflammatory chemokine secretion in intestinal epithelial cells: implications for specific IBD indications.", JOURNAL OF CLINICAL GASTROENTEROLOGY SEP 2010 LNKD- PUBMED:20562631, vol. 44 Suppl 1, September 2010 (2010-09), pages S10-S15, XP009147284, ISSN: 1539-2031

## Description

The invention relates to a novel approach to prevent or treat inflammatory and/or allergic diseases and, in particular, IP-10-mediated inflammatory diseases and/or Eotaxin-mediated allergic reactions through the use of bacterial proteases belonging to the group of lactocepins. According to the invention, a completely new and unexpected property of lactocepins has been found, i.e., their ability to specifically target a specific subset of pro-inflammatory cytokines and chemokines, in particular IP-10 and/or Eotaxin. This property of lactocepins has opened up a new therapeutic field to this effect that inflammatory diseases can be treated selectively and essentially without the risk of serious side effects. The use and the treatment, respectively, according to the present invention is particularly suitable for the treatment of inflammatory bowel diseases.

Inflammatory diseases are acute and chronic diseases by which inflammatory cells and inflammatory substances of the body's immune system cause series of symptoms including redness, swelling, heat and tissue damage. In particular, chronic inflammatory diseases may lead to the destruction of important tissue or organ structures of the body. Such diseases are difficult to treat or to cure. Mostly, said diseases are treated by the use of immune-suppressant drugs, which treatment is usually associated with serious side effects.

Inflammatory bowel diseases (IBD) are spontaneously relapsing immunologically-mediated disorders of the gastrointestinal tract. The incidence and prevalence of the two main idiopathic pathologies of IBD, Crohn's disease (Morbus Crohn) and ulcerative colitis (colitis ulcerosa) are rising globally in parallel to the progression of the industrialization. So far, chronic inflammatory bowel diseases are treated by means of a symptomatic therapy, for example, by use of non-steroidal cyclooxygenase 2 inhibitors (COX 2 inhibitors) such as mesalacine and its derivatives, steroidal anti-inflammatory substances like cortisone or immunosuppressants. Said therapies of the art are regularly associated with frequent and strong side effects as well as a low or lacking efficiency in some patient populations. More recent therapeutic approaches in the treatment of chronic inflammatory bowel diseases concern the selective neutralisation of the main inflammatory cytokine, tumor necrosis factor alpha (TNF alpha), by means of anti-TNF antibodies (for example Infliximab). Said therapy, however, leads to strong side effects such as a decreased resistance to infections or autoimmune reaction due to the systemic weakening of the immune system through the systemic administration (infusion). Infliximab may, therefore, only be used with patients having not successfully reacted on other therapies.

Consequently, there is a need in the art for an effective therapy of chronic inflammatory diseases with no or only minor side effects. It is, therefore, an object of the present invention to provide a therapeutic approach in the treatment of inflammatory diseases which is therapeutically efficient, well tolerable to the patient and essentially free of side effects. The new therapeutic approach shall be suitable for a wide variety of acute and chronic inflammatory diseases, easy to apply and suitable for long-term treatment. The therapeutic approach according to the invention should be suitable for establishing, maintaining or restoring a healthy immune function and should be well tolerable in terms of acute or systemic side effects.

The present invention is based on the unexpected and surprising finding that the bacterial proteases of the lactocepin group exert a therapeutic effect locally at the side of an inflammation, particularly an intestinal inflammation, thus selectively reducing the incidence of inflammation essentially without side effects.

It has been found that lactocepins and in particular lactocepins isolated from probiotic bacteria have the ability to degrade selectively a variety of inflammation-related cytokines and chemokines, in particular, IP-10 and/or Eotaxin.

Consequently, the present invention relates to lactocepin and/or a functionally active fragment thereof for use in the prophylaxis and/or the treatment of a disease. In particular, the present invention relates to one or more lactocepin(s) and/or one or more functionally active fragment(s) thereof for use in the prophylaxis and/or treatment of an inflammatory disease and preferably of IP-10-mediated inflammatory diseases.

Further, the present invention relates to one or more lactocepin(s) and/or one or more functionally active fragment(s) thereof for use in the prophylaxis and/or treatment of an allergic reaction or disease and preferably of an Eotaxin-mediated allergic reaction or disease.

The disclosure moreover, relates to the use of one or more lactocepin(s) and/or one or more functionally active fragment(s) thereof for the preparation of a pharmaceutical or therapeutic nutritional composition for the treatment and/or prophylaxis of an inflammatory disease and preferably of IP-10-mediated inflammatory diseases or of an allergic reaction and preferably of an Eotaxin-mediated allergic reaction.

The disclosure further concerns a method of treatment and/or prophylaxis of inflammatory diseases by administering one or more lactocepin(s) and/or one or more functionally active fragment(s) thereof to a subject suffering from an inflammatory disease, in particular, from an IP-10-mediated inflammatory disease.

The disclosure further concerns a method of treatment and/or prophylaxis of inflammatory diseases by administering one or more lactocepin(s) and/or one or more functionally active fragment(s) thereof to a subject suffering from an allergic reaction, in particular, from an Eotaxin-mediated allergic reaction.

The invention further concerns a pharmaceutical preparation comprising one or more lactocepin(s) and/or one or more functionally active fragment(s) thereof. Further, the invention relates to a pharmaceutical composition comprising one or more lactocepin(s) and/or one or more functionally active fragment(s) thereof for use in the prophylaxis and/or treatment of an inflammatory disease, in particular, of an IP-10-mediated inflammatory disease.

Further, the invention relates to a pharmaceutical composition comprising one or more lactocepin(s) and/or one or more functionally active fragment(s) thereof for use in the prophylaxis and/or treatment of an allergic reaction, in particular, of an Eotaxin-mediated allergic reaction.

The endopeptidase lactocepin is a protease known from various lactic acid bacteria such as *Lactobacillus acidophilus*, *Lactobacillus lactis* etc. So far, for lactocepin no therapeutic use and, in particular, no therapeutic use in the treatment of inflammatory diseases is known from the art.

Lactocepins were first discovered in *Lactococcus lactis* and caseins were found to be the main substrate of this bacterial protease. In consequence, lactocepin-expressing bacteria are capable of using caseins for their amino acid supply (Juillard et al., 1995, "The extracellular PI-type proteinase of Lactococcus lactis hydrolyzes beta-casein into more than one hundred different oligopeptides", J Bacteriol 177, 3472-8). There are three known types of lactocepins, PI to PIII, which show at least 95% sequence homology. The different lactocepin types are classified according to biochemical properties and according to their specificity towards casein subgroups. These subtypes can be subclassified into different groups of lactocepins (a-f) according to their preferred cleavage sites of the substrate (Holck and Naes, 1992, "Cloning, sequencing and expression of the gene encoding the cell-envelope-associated proteinase from Lactobacillus paracasei subsp. paracasei NCDO 151" J Gen Microbiol 138, 1353-64.). Apart from the respective amino acid sequence of the proteases, the specificity of lactocepins towards alpha, beta or kappa casein was found to be determined via post-translational modifications, mainly through autoproteolysis (Flambard and Juillard, 2000, "The autoproteolysis of Lactococcus lactis lactocepin III affects its specificity towards beta-casein" Appl Environ Microbiol 66, 5134-40). Genetic engineering experiments revealed that lactocepin is still active even if large peptide sequences are excised (Bruinenberg et al., 1994, "Evidence for a large dispensable segment in the subtilisin-like catalytic domain of the Lactococcus lactis cell-envelope proteinase", Protein Eng 7, 991-6).

EP 0 555 618, US 6,544,568, US 5,716,615 and US 2005/0186190 disclose dietetic and/or pharmaceutical compositions containing lyophilized lactic bacteria.

In the art it has generally been known that probiotic bacterial strains like *Escherichia coli* Nissle 1917 or the ones in VSL#3 have a favourable effect on the pathology of inflammatory bowel diseases (see G. Hörmannsperger, "Post-Translational Inhibition of IP-10 Secretion in a IEC by Probiotic Bacteria: Impact on Chronic Inflammation", PLoS One, 4, e4365, 2009). It has particularly been shown that the probiotic mixture VSL.#3, a mixture of eight different lactic acid bacteria (*Lactobacillus (L.) acidophilus, L. bulgaricus, L. paracasei, L. plantarum, Streptococcus thermophilus, Bifidobacterium (B.) breve, B. infantis, B. longum*) was effective in the prevention and in the maintenance treatment of pouchitis and ulcerative colitis. The favourable effect of said mixture on said diseases, however, could neither be attributed to a specific compound nor could it be standardized.

The favourable effects of said probiotic mixture was seen in a complete inhibition of IP-10 protein secretion in IEC, resulting in inhibited IP-10-mediated T cell transmigration. On the basis of the above reference it was believed that *L*. *paracasei* impairs the secretory machinery important for the secretion of IP-10 in IEC and that this secretory blockade was followed by subsequent intracellular degradation of the pro-inflammatory chemokine. Chemokines are secreted by the cells as an accessory signal to attract pro-inflammatory immune cells along the chemokine gradient to the side of secretion. Said chemokine gradient ranges from the secreting cell through parts of the tissue into the blood. When an inflammatory disease is diagnosed, the chemokine gradient is already existent and inhibition of the secretion of new chemokines has no or only a protracted influence on that proinflammatory chemokine gradient. On the basis of the prior art, probiotic bacteria were assumed to induce a blockade of IP-10 secretion in one specific cell type, intestinal epithelial cells, thus limiting the application locally.

Contrary to that finding of the prior art, the present inventors have now found out that the favourable effect of said mixture of bacteria is due to the effect of one or more specific protease(s) which is expressed and/or secreted by probiotic bacteria and which belongs to the group of lactocepins. That protease has been identified by means of liquid chromatography-mass spectrometry-mass spectrometry (LC-MS-MS) analysis of active chromatographic fractions as lactocepin. The lactocepins have been found to have the unexpected ability to degrade chemokines, in particular IP-10, independent of the cellular source of the chemokines. This means that lactocepin is able to degrade chemokines and, in particular, IP-10 in all tissues and body fluids (such as blood, articular liquid etc.) which opens up a large therapeutic field for lactocepins in relation to a wide variety of inflammatory diseases. The direct degradation of chemokines and, in particular, of IP-10 by use of lactocepin is a basic requirement for its effect in peritoneal, in-joint, intravenous or cutaneous applications. According to the prior art, only the oral administration of said at that time unidentified probiotic compound would have been supposed to result in a delayed reduction of tissue IP-10 levels via the induction of a secretory blockade in intestinal epithelial cells. This means that according to the prior art only an application in gut-associated diseases would have been expected, if at all. The inflamed tissue of IBD patients is very often characterized by highly damaged or high loss of intestinal epithelial cells. According to the prior art, the use of a bacterial serine protease would not have been expected to be effective in this case, since the induction of a secretory blockade for IP-10 in IEC is dependent on the existence of responsive intestinal epithelial cells. In contrast, the direct degradation of IP-10 via orally or peritoneally administered lactocepin is independent of intestinal epithelial cell functions. Since a directly acting lactocepin may penetrate into the deep intestinal layers of the tissue and destroy even pre-established chemokine gradients independent of the cellular source, intestinal inflammatory diseases may be effectively cured. This means that on the basis of the inventors' finding lactocepins may be used in the treatment of various kinds of inflammatory diseases.

Said finding must be deemed surprising since on the basis of the art it had to be expected that probiotic bacteria such as *L. paracasei* induced intracellular protective mechanisms in intestinal epithelial cells (IEC). It could not have been expected that *L. paracasei* secretes a protease with anti-inflammatory properties due to the selective substrate specificity of this protease, lactocepin, particularly for IP-10.

Ex vivo incubation of stretches of inflamed tissue with lactocepin-containing medium (*L. paracasei* (VSL#3)-conditioned medium) led to a significant reduction of IP-10 in the tissue supernatants whereas the amount of secreted tumor necrosis factor was not influenced. It has been shown that the selective reduction of IP-10 in a medium supporting inflammation is sufficient to prevent trafficking of immune cells. It has further been shown that lactocepins have a broad but variable substrate spectrum towards inflammatory cytokines.

While lactocepins are highly active in degrading IP-10, they also degrade the following cytokines (m = murine, h = human):
mIP-10 (CXCL10), m-ITAC (CXCL11), hIP-10, hITAC (2 of 3 CXCR3-targeting chemokines)
hSDF-1α, mSDF-1α, hFractalkine, hTeck (CCL25), hEotaxin.

The following cytokines are not degraded by lactocepins:
mIL-6, mTNF (cytokines)
hIL-8 (CXCL8), mRANTES(CCL5), hMIP-3α (CCL20), mMIP-2α (CXCL2) (chemokines)

Accordingly, one or more lactocepin(s) and/or one or more functionally active fragment(s) thereof may also be used in the prophylaxis and/or treatment of an Eotaxin-, ITAC-, SDF-1α-, Fractalkine- or/and Teck-mediated inflammatory disease.

Eotaxin plays an important role in allergic reactions. Thus, lactocepin(s) and/or functionally active fragments thereof can particularly be used in the prophylaxis and/or treatment of allergic reactions and, in particular, of inflammatory allergic reactions.

Said findings reveal degradation of IP-10 as a new probiotic mechanism and as a new function of lactocepins. This anti-inflammatory effect of VSL#3 was found to be mediated by *L. paracasei*-derived lactocepin and was proven to be of relevance in specific inflammatory bowel disease indications *in vivo.* The inhibitory effect of VSL#3 on IP-10 expression was revealed to be due to a single bacterial strain of the probiotic mixture *L. paracasei* (VSL#3). The protective bacterial structure of *L. paracasei* (VSL#3) was found to be a bacterial serine protease and was identified as lactocepin type II (PrtP). Lactocepins are bacterial cell surface proteases which can also be shaded off the membrane via autoproteolysis explaining why both whole cell *L*. *paracasei* (VSL#3) as well as bacterial supernatants were found to result in loss of IP-10. According to the invention it has thus been shown that lactocepin is one of the very first probiotic structures that exert a specific anti-inflammatory effect.

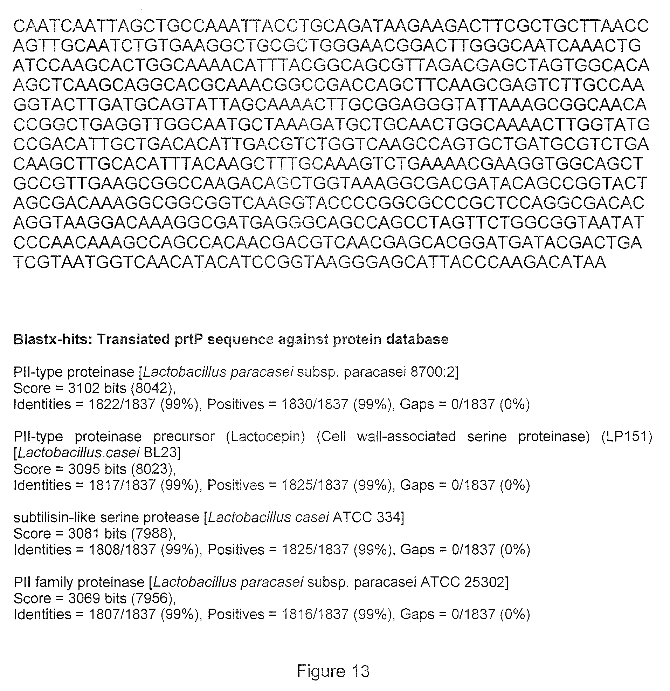

The DNA sequence of lactocepin from L.p VSL#3 strain without the secretion sequence is shown in Fig. 13 and as SEQ ID NO: 1. Preferably, a lactocepin is used which has an identity of at least 50%, in particular, of at least 60%, preferably of at least 80%, more preferably at least 90% and most preferably at least 95% with the lactocepin encoded by SEQ ID NO: 1.

It was found that the protein sequence encoded by SEQ ID NO: 1 has an identity of 99% with PII-type proteinase of *Lactobacillus paracasei,* an identity of 99% with PII-type proteinase precursor (lactocepin) of *Lactobacillus casei* BL23, an identity of 99% with subtilisin-like serine protease of *Lactobacillus casei* ATCC334 and an identity of 99% with PII-family proteinase of *Lactobacillus paracasei* ATCC25302.

It has been found that the lactocepin gene is not only present in *L. paracasei* (VSL#3) but also in other *L. casei* strains such as BL23 and ATCC334. This means that not only *L. paracasei* VSL#3 but also other probiotic bacteria containing (and expressing) the lactocepin gene are able to exert the lactocepin-related anti-inflammatory effect. The actual expression of lactocepin depends on the growth conditions used.

From the above results it follows that lactocepin is an IP-10-specific protease by means of which inflammatory diseases and, in particular, chronic inflammatory bowel diseases, can be treated essentially without the risk of serious side effects.

Since IP-10 is fundamentally involved in a wide variety of inflammatory diseases, the following diseases are susceptible to a treatment with lactocepins:
- Rheumatoide Arthritis (Kuan, W. P., L. S. Tam, et al. (2009). "CXCL 9 and CXCL 10 as Sensitive Markers of Disease Activity in Patients with Rheumatoid Arthritis." J Rheumatol, online 23.12.09, ahead of print,
- Different types of arthritis (Antonelli, A., P. Fallahi, et al. (2009). "High values of Th1 (CXCL10) and Th2 (CCL2) chemokines in patients with psoriatic arthtritis." Clin Exp Rheumatol 27(1): 22-7)
- Arthritis and bone erosion (Kwak, H. B., H. Ha, et al. (2008). "Reciprocal cross-talk between RANKL and interferon-gamma-inducible protein 10 is responsible for bone-erosive experimental arthritis." Arthritis Rheum 58(5): 1332-42)
- Wound healing after spinal cord injury (Glaser, J., R. Gonzalez, et al. (2004). "Neutralization of the chemokine CXCL10 enhances tissue sparing and angiogenesis following spinal cord injury." J Neurosci Res 77(5): 701-8)
- Multiple sclerosis (Balashov, K. E., J. B. Rottman, et al. (1999). "CCR5(+) and CXCR3(+) T cells are increased in multiple sclerosis and their ligands MIP-1alpha and IP-10 are expressed in demyelinating brain lesions." Proc Natl Acad Sci U S A 96(12): 6873-8)
- Different autoimmune diseases (Lee, E. Y., Z. H. Lee, et al. (2009). "CXCL10 and autoimmune diseases." Autoimmun Rev 8(5): 379-83)
- Autoimmune-or virus-mediated hepatitis (Nishioji, K., T. Okanoue, et al. (2001). "Increase of chemokine interferon-inducible protein-10 (IP-10) in the serum of patients with autoimmune liver diseases and increase of its mRNA expression in hepatocytes." Clin Exp Immunol 123(2): 271-9)
- Lupus erythematosus (Narumi, S., T. Takeuchi, et al. (2000). "Serum levels of ifn-inducible PROTEIN-10 relating to the activity of systemic lupus erythematosus." Cytokine 12(10): 1561-5)
- Chronic inflammation in obesity and artherosclerosis (Laine, P. S., E. A. Schwartz, et al. (2007). "Palmitic acid induces IP-10 expression in human macrophages via NF-kappaB activation." Biochem Biophys Res Commun 358(1): 150-5)
- Graft-versus-host disease and graft-dysfunction/rejection (Piper, K. P., C. Horlock, et al. (2007). "CXCL10-CXCR3 interactions play an important role in the pathogenesis of acute graft-versus-host disease in the skin following allogeneic stem-cell transplantation." Blood 110(12): 3827-32)
- Artherosclerosis and coronary heart disease (Zernecke, A., E. Shagdarsuren, et al. (2008). "Chemokines in atherosclerosis: an update." Arterioscler Thromb Vasc Biol 28(11): 1897-908.

Also the above further chemokines, which are degraded by lactocepin, are related to serious diseases. Therefore, the following further diseases may also be treated using lactocepin. According to Odai, T., M. Matsunawa, et al. (2009), "Correlation of CX3CL1 and CX3CR1 levels with response to infliximab therapy in patients with rheumatoid arthritis." J Rheumatol 36(6): 1158-65, fractalkines (Cx3CL1) are involved in the pathology of arthritis. Fractalkines and ITAC are involved in the pathology of atherosclerosis (Zernecke, A., E. Shagdarsuren, et al. (2008), "Chemokines in atherosclerosis: an update." Arterioscler Thromb Vasc Biol 28(11): 1897-908), I-TAC are involved in transplant rejection (Li, B., W. Xu, et al., "I-TAC is a dominant chemokine in controlling skin intragraft inflammation via recruiting CXCR3+ cells into the graft." Cell Immunol 260(2): 83-91).
Further to this, CXCL10, fractalkine, CCL20 and CCL25 are of importance in the pathology of inflammatory bowel diseases.

The lactocepin(s) and/or functionally active fragment(s) thereof are preferably used for the prophylaxis or treatment of a disease selected from chronic and acute inflammations, autoimmune diseases, inflammatory bowel diseases (IBD), in particular, Crohn's disease (Morbus Crohn) or/and ulcerative colitis (colitis ulcerosa), arthritis, in particular, rheumatoid arthritis, psoriatic arthritis, bone erosion, wound healing after spinal cord injury, multiple sclerosis, autoimmune diseases, hepatitis, in particular, autoimmune- or virus-mediated hepatitis, lupus erythematosus, atherosclerosis, graft-versus-host disease or graft-dysfunction/rejection and coronary heart diseases.

It is further described that the lactocepin or a functionally active fragment thereof may be present in a suitable bacterial host. This may be bacteria that naturally express lactocepin or bacteria which have been modified to express or overexpress lactocepin. Mixtures of bacteria expressing lactocepin optionally together with other probiotic bacteria may as well be used. Examples of corresponding bacteria are probiotic bacteria, lactic acid bacteria or Bifidobacteria with established probiotic characteristics. Examples of corresponding bacteria are *Lactobacillus acidophilus, Lactobacillus bulgaricus, Lactobacillus casei, Lactobacillus plantarum, Streptococcus thermophilus, Bifidobacterium breve, Bifidobacterium infantis, Bifidobacterium longum* or the probiotic mixture VSL#3, or enterococci such as *E. coli.* The lactocepin gene may be cloned into the following hosts *Lactobacillus acidophilus, Lactobacillus bulgaricus, Lactobacillus casei, Lactobacillus plantarum, Streptococcus thermophilus, Bifidobacterium breve, Bifidobacterium infantis, Bifidobacterium longum, Escherichia coli, Lactococcus lactis.*

Preferably, the probiotic strain suitable as a host is *L. paracasei* (VSL#3).

A person skilled in the art is well in a position to genetically modify bacteria to express or over-express lactocepin. Corresponding techniques are known in the art.

The term "lactocepin" preferably denotes lactocepin type 1 or 2 (PRTP), but denotes any other lactocepins that have the ability to degrade IP-10. A person skilled in the art is well in a position to determine whether a lactocepin, i.e., a compound belonging structurally to the group of lactocepins has the ability to degrade IP-10.

The term "functionally active fragment of lactocepin" is to denote any fragment of lactocepin that still has the ability to degrade IP-10. Preferably, that functionally active fragment is the smallest still active fragment of lactocepins in order to increase tissue penetration. A functionally active fragment of lactocepin may also be combined with non-lactocepin sequence parts provided that the activity of the whole molecule to degrade IP-10 is still maintained.

The fragment may have a length of at least 100 amino acids, preferably at least 200 amino acids and more preferably at least 500 amino acids. It has been found that the three amino acids forming the active center for the degradation of IP-10 are about 20 amino acids apart from each other.

Lactocepin, a functionally active fragment thereof and lactocepin-expressing bacteria may be formulated into a pharmaceutical or a therapeutic nutritional composition by means of suitable excipients. The pharmaceutical composition comprises a therapeutically effective amount of lactocepin and/or a functionally active fragment thereof and/or at least one lactocepin-expressing bacterial strain and can take the form of tablets, capsules, liquid bacterial suspensions, dried oral supplements, wet oral supplements, dry tube feeding or wet tube feeding.

The pharmaceutical composition may be for oral, parenteral, topical, nasal, ocular, rectal, intravenous, intraperitoneal or local in-joint administration. Preferably, the pharmaceutical composition is in a form for oral, parenteral, topical, in-joint or cutaneous administration. Said compositions are formulated using excipients and formulations well known in the art for corresponding preparations. One or more drugs supporting the intended therapeutic effect may be incorporated into the compositions.

The drugs to be further included in such a pharmaceutical composition may be any drug that is physiologically compatible with lactocepin. Examples of drugs that may be combined with lactocepins are anti-convulsant, anticholinergic, anti-histaminic, adrenergic, sedative, anti-inflammatory, antipyretic, antiseptic, analgesic, anti-rheumatic, diuretic, anti-psychotic, antibacterial, hepato-protector, anti-lipemic drugs and analeptic drugs. It is preferred to combine lactocepine with anti-inflammatory drugs such as corticosteroids, non-steroidal anti-inflammatory drugs such as Mesalazine, Infliximab, immune-suppressiva. Moreover, vitamins, trace elements, secondary plant products, amino acid and probiotic bacteria may also be used in combination with lactocepin.

The pharmaceutical composition may be formulated according to usual pharmaceutical forms known in the art. Examples are tablets, coated tablets, capsules, packages, solutions, suspensions, emulsions, suppositories, pellets, syrups, vaginal suppositories, ointments or creams.

The composition according to the present invention may also be in the form of a therapeutic nutritional composition comprising either lactocepin and/or a functionally active fragment thereof and/or at least one lactocepin-expressing bacterial strain. Non-limiting examples of suitable nutritional composition which can be used within the scope of the present invention are milk, milk products such as yoghurt, cheese, curd, fermented milks, milk-based fermented products, fermented cereal-based products, fermented milk products, milk-based or cereal-based powders, clinical nutrition formulae, ice creams, juices, bread, cakes, candies, animal feed formulations, semi- or synthetic diet formulations or infant formulations. If necessary and desired, said formulations may contain further ingredients such as other probiotic bacteria, a source of fat, a source of carbohydrate, a source of protein as well as vitamins/minerals and micronutrients. Said compositions may further contain prebiotic ingredients, emulgators, flavouring agents, preservatives, stabilizers, emulsifying agents, buffers, colorants, antioxidants, antimicrobials, bulking agents and the like. A person skilled in the art will be in a position to select a suitable composition having regard to the kind of composition as desired.

The composition of the invention may also be in the form of a feed, in particular, for administration to animals.

The required dosage amount of lactocepin, and/or a functionally active fragment thereof and/or lactocepin-expressing bacteria in the pharmaceutical or therapeutic nutritional compositions for use according to the invention will vary according to the nature of the inflammatory disease to be treated or the purposed use of the composition, i.e., whether used prophylactically or therapeutically, and the type of organism involved.

Any suitable dosage of lactocepin, and/or a functionally active fragment thereof and/or lactocepin-expressing bacteria may be used that has a prophylactic and/or therapeutic effect. In case of a pharmaceutical composition, the dosis of lactocepin is in the range of 70 to 500 mg/day, preferably 100 to 400 mg/day, more preferably 150 to 300 mg/day. Preferably the dosage is taken in three portions daily, preferably before a meal.

The exact dosage to be administered varies within the above range depending on the dosage form employed, the sensitivity of the patient and the route of administration. The exact dosage will be determined by the practitioner in light of factors related to the subject requiring treatment.

As regards a therapeutic nutritional composition for use according to the present invention, the amount of lactocepin in that composition varies from 200 to 400 mg per portion.

According to the present invention, lactocepin, a functionally active fragment thereof and/or lactocepin-expressing bacteria are preferably used to treat inflammatory bowel diseases and, in particular, Morbus Crohn (Crohn's disease), Colitis ulcerosa (ulcerative colitis) and pouchitis.

Although probiotic bacteria are well known in wellness and convenience products, the scientific literature is completely silent as to the existence of the kind of an active component being responsible for their general positive effect on the human health. The reason for this fact is that it is basically not easy to isolate and to identify a specific compound out of that complex mixtures of compounds contained in probiotic bacteria (for example secreted compounds, lysed bacteria, membrane-bound compounds). So far, only the flagellum of *E. coli* Nissle 1917 as well as P40/P75 of *Lactobacillus rhamnosus* G.G. have been identified as active principle in probiotic bacteria. Lactocepin is thus the third active compound ever isolated from probiotic microorganisms.

To identify lactocepin, it was necessary to devise a specific chromatographic separation not only to isolate specific compounds but to isolate specific active compounds out of the bacteria. This means that specific chromatographic and elution conditions had to be established. Due to the high dissolution of the active compound after the chromatographic separation, all fractions had to be concentrated before they could be tested on their activity. The probiotic activity was then found in many separated fractions. That unexpected result may be due to several active compounds such as proteases, active oligomers or partly digested active compounds. A comparison of the active fractions on SDS gels did not lead to a conclusive result since the active fractions showed several but not conforming protein bands. Only a LC-MS-MS comparison of active and non-active chromatographic fractions of *L*. *paracasei* provided the information that in all active fractions lactocepin fragments were present.

After the isolation of the active compound the receptor or target protein of lactocepin on intestinal epithelial cells had to be found. It was completely unexpected when incubation experiments with bacterial supernatants of *L*. *paracasei* and IP-10 enriched supernatants of activated epithelial cells showed that the bacterial supernatant contains compounds which surprisingly led to a selective loss of IP-10 in the absence of intestinal epithelial cells. To find out which bacterial compound was responsible for that effect, several experiments using recombinant IP-10 were carried out: Incubation of recombinant IP-10 with bacterial supernatant or fixed *L*. *paracasei* VSL#3 led to a total degradation of IP-10 (see experimental section). The supernatants of *L. casei* BL23 and 334 were found to lack lactocepin and did not lead to a loss of recombinant IP-10. Treatment with PMSF (a serine protease inhibitor) of the bacterial supernatant inhibited the degradation. It was then found that the active compound has a molecular weight greater than 100 kDa.

On the basis of these experiments it was found that lactocepin is the active compound that is able to degrade IP-10 as well as other chemokines independent of any cellular compounds.

The disclosure concerns in particular the following items:
1. Lactocepins and/or a functionally active fragment thereof for use in the prophylaxis and/or the treatment of a disease.
2. One or more lactocepin(s) and/or one or more functionally active fragment(s) thereof for use in the prophylaxis and/or treatment of IP-10-mediated inflammatory diseases.
3. The lactocepin(s) and/or functionally active fragment(s) thereof for use according to item 2, wherein the lactocepin(s) and/or the functionally active fragment(s) thereof is/are comprised in a suitable bacterial host.
4. The lactocepin(s) and/or functionally active fragment(s) thereof for use according to one of items 2 or 3, wherein the lactocepin(s) and/or functionally active fragment(s) thereof are comprised in a pharmaceutical or a therapeutic nutritional composition.
5. The lactocepin(s) and/or functionally active fragment(s) thereof for use according to one of items 2 to 4, wherein the composition further comprises one or more ingredients selected from physiologically compatible drugs, dietary supplements or pharmaceutically or physiologically acceptable excipients.
6. The lactocepin(s) and/or functionally active fragment(s) thereof for use according to one of items 3 to 5, wherein the bacterial host is selected from bacteria naturally expressing lactocepin or a functionally active fragment thereof or bacteria genetically modified to express or over-express lactocepin or a functionally active fragment thereof.
7. The lactocepin(s) and/or functionally active fragment(s) thereof for use according to one of items 2 to 6, wherein the lactocepin is lactocepin derived from *L. paracasei* (VSL#3) or *Lactococcus lactis cremoris* SK11.
8. The lactocepin(s) and/or functionally active fragment(s) thereof for use according to one of items 2 to 7, wherein the IP-10-mediated inflammatory disease is selected from inflammatory bowel diseases, autoimmune diseases, chronic or acute inflammations, atherosclerosis, arthritis or coronary heart disease.
9. The lactocepin(s) and/or functionally active fragment(s) thereof for use according to one of items 4 to 8, wherein the pharmaceutical composition is in a form for oral, parenteral, topical, in-joint or cutaneous administration.
10. The lactocepin(s) and/or functionally active fragment(s) thereof for use according to one of items 4 to 8, wherein the therapeutic nutritional composition is in the form of a milk product.
11. Use of one or more lactocepin(s) and/or one or more functionally active fragment(s) thereof for the preparation of a pharmaceutical or therapeutic nutritional composition for the treatment and/or prophylaxis of IP-10-mediated inflammatory diseases.
12. The use according to item 11, wherein the lactocepin(s) and/or the functionally active fragment(s) thereof is/are comprised in a suitable bacterial host.
13. The use according to one of items 11 or 12, wherein the composition further comprises one or more ingredients selected from physiologically compatible drugs, dietary supplements or pharmaceutically or physiologically acceptable excipients.
14. The use according to one of items 12 or 13, wherein the bacterial host is selected from bacteria naturally expressing lactocepin or a functionally active fragment thereof or bacteria genetically modified to express or over-express lactocepin, or a functionally active fragment thereof.
15. The use according to one of items 11 to 14, wherein the lactocepin is derived from *L. paracasei* (VSL#3) or *Lactococcus lactis cremoris* SK11.
16. The use according to one of items 11 to 15, wherein the IP-10-mediated inflammatory disease is selected from inflammatory bowel diseases, autoimmune diseases, chronic or acute inflammations, atherosclerosis, arthritis or coronary heart disease.
17. The use according to one of items 11 to 16, wherein the pharmaceutical composition is in a form for oral, parenteral, topical, in-joint or cutaneous administration.
18. The use according to one of items 11 to 16, wherein the therapeutic nutritional composition is in the form of a milk product.

The following figures explain the subject-matter of the present invention:
Figure 1:
   The active secreted component of *L. paracasei* (VSL#3) (L.c) is a bacteria protein. ELISA analysis with differently treated CM *L. paracasei* (VSL#3) revealed that heating of CM *L. paracasei* (VSL#3) abrogates its inhibitory activity on IP-10 secretion, whereas 50% ammonium sulfate precipitation results in complete precipitation of the active component. Activity is regained by resolubilisation of the precipitate whereas the precipitate supernatant does not exert any detectable activity on IP-10 secretion.
Figure 2:
   The active secreted bacterial protein is bigger than 50 kDa. Concentration of CM *L. paracasei* (VSL#3) using a 50 kDa filter device results in loss of activity in the flow-through whereas the retentate (1x) remains active.
Figure 3:
   The active secreted protein of *L. paracasei* (VSL#3) is a bacterial serine protease. The application of the irreversible serine protease inhibitor PMSF completely abrogates the inhibitory effect of CM *L. paracasei* (VSL#3) on IP-10 protein secretion (upper panel) and rescues intracellular IP-10 protein (lower panel) (A). The observed inhibition of IP-10 degradation is due to the inhibition of a bacterial protease and not due to the inhibition of a cellular protease, as PMSF-preincubation (1 h) of Mode K cells does not inhibit subsequent induction of IP-10-degradation via CM *L. paracasei* (VSL#3) (B).
Figure 4:
   *L. paracasei* (VSL#3), but not *L. casei* BL23 secretes a bacterial serine protease which is able to degrade FTC-casein. CM *L. paracasei* (VSL#3) (10x) is able to degrade FTC-casein whereas CM *L. casei* BL23 (10x) does not show proteolytic activity.
Figure 5:
   The anti-inflammatory bacterial protease secreted by *L*. *paracasei* (VSL#3) does not negatively affect TEER. The stimulation of T84 cells with CM *L*. *paracasei* (VSL#3) and CM *L*. *casei* BL23 revealed that the active bacterial protease in the CM *L. paracasei* (VSL#3) does not affect normal or TNF/IFNγ-reduced TEER.
Figure 6:
   Chromatographic fractionation of CM *L. paracasei* (VSL#3) results in several separated active fractions. Chromatographic separation of the constituents of CM *L. paracasei* (VSL#3) according to molecular mass (Superdex), charge (Mono-Q) or hydrophobicity (Phenyl-Sepharose) was performed. Subsequent cell culture stimulation experiments revealed probiotic activity in several chromatographic fractions which were often separated by non-active fractions.
Figure 7:
   IL-10-I- mouse colonic explant secretion (inflamed tissue sections (24 hours).
   The following examples further illustrate embodiments of the invention:
Figure 8:
   IP-10 as direct substrate of lactocepin of *L*. *paracasei.*
Figure 9:
   IP-10 as direct substrate of lactocepin of *L. paracasei* vs. *L. casei* BL23.
Figure 10:
   Degradation of chemokines by the proteolytic action of lactocepin of *L*. *paracasei.*
Figure 11:
   Selective degradation of IP-10 vs. IL-6.
Figure 12:
   Results of an experiment using conditioned medium (CM) of *Lactobacillus paracasei* of VSL#3.
Figure 13:
   DNA sequence (SEQ ID NO: 1) of lactocepin from L.p VSL#3 strain without secretion sequence.

### Examples

### Example 1 (Referential Example):

### Material and Methods

**Bacterial culture and treatment.** Lyophilized VSL#3 bacteria (a generous gift from Dr. DeSimone, L'Aquila, Italy) were resuspended in DMEM before they were used in cell culture stimulation experiments. VSL#3 derived *L*. *casei* (L.c) (reclassified as *L. paracasei* according to VSL#3 pharma in 2008) (a generous gift from Dr. DeSimone, L'Aquila, Italy), *L. casei* BL23, *L*. *plantarum* 299v (generous gifts from Dr. Gaspar, Instituto de Agroquimica y Tecnologia de Alimentos, Valencia, Spain) and *L. casei* ATCC393 were grown at 37°C in MRS Broth (Fluka, Heidelberg) containing 0.05% L-cysteine (Roth) under anaerobic conditions using Anaerogen packages (Anaerogen, Oxoid, UK). *E. coli* strain Nissle 1917 (a generous gift from Dr. Sonnenborn, Ardeypharm GmbH, Herdecke, Germany) was grown aerobically in LB-medium (AppliChem, Darmstadt, Germany). Bacteria were centrifuged (4500 g, 10 min), washed in an equal volume of PBS (1x) (4500 g, 10 min) and resuspended in DMEM. To characterize effective probiotic components, *L*. *paracasei* was heat-killed (90°C, 30 min), fixed (fL.c) (5% formaldehyde, 3 h, 4°C) or lysed by lysozyme (50 mg/ml) (Sigma, Steinheim, Germany) in filter sterilized Tris buffer (10 mM Tris, pH8). Fixed bacteria were washed three times with sterile 1xPBS (Invitrogen, Carlsbad, USA) and resuspended in the respective cell culture media. All cell culture experiments were performed with fixed bacteria, if not otherwise indicated. For cell surface treatment, bacteria were incubated with Phospholipase A (2 mg/ml) (Sigma, Steinheim, Germany), Trypsin (2 mg/ml) (Roth, Karlsruhe, Deutschland), Proteinase K (50 µg/ml) (Roth, Karlsruhe, Deutschland) in filter sterilized Tris buffer (50 mM Tris, 0,1 M NaCl, pH 8) (37°C, 1 h) in a shaker.

**Preparation of bacterial conditioned media (CM).** CM was generated by growing VSL#3-derived *L. paracasei*, *L. casei* BL23, *L. casei* ATCC393 and *L. plantarum* 299v (5x10⁷ cfu/ml) anaerobically in DMEM (1% Glutamine) containing HEPES (20 mM) (Invitrogen, Carlsbad, USA) (37°C, 5% CO₂, 24 h). CM was cleared from bacterial cells (4500 rpm, 10 min), titrated to a pH of 7,6 and filter-sterilized. Sterile CM was either supplemented with FCS and used to replace the cell culture media in cell culture stimulation experiments or concentrated using 10 or 50 kDa amicon filter devices (Millipore, Carrigtwohill, Ireland) and diluted to 1xCM in the cell culture supernatant. Where indicated, CM was heat-inactivated (60°C, 20 min), supplemented with PMSF (1 mM) (RT, 15 min) or underwent precipitation with 50% ice cold ammonium sulfate (-20°C, o.n.) followed by resuspension of the dried precipitate (12000g, 4°C, 10 min) in DMEM.

**Protease Assay.** Serin/cystein protease activity of CM *L. paracasei* (VSL#3) and CM *L. casei* BL23 was analysed using a Protease Assay Kit (Cat. Nr. 539125, Calbiochem, Darmstadt, Germany) which is based on proteolytic degradation of FTC-casein according to the manufacturers instructions.

**Cell culture stimulation.** Confluent Mode-K or HEK cell monolayers were stimulated with TNF (10 ng/ml) (R&D Europe, Abington, England), IFNγ (50 ng/ml), brefeldin A (0.5 µM) (Calbiochem, Darmstadt, Deutschland), lactacystin (22 mM) (Biomol, Hamburg, Germany), NH₄Cl (20 mM) (Sigma, Steinheim, Germany), 3-methyladenine (3-MA) (5 mM) (Sigma, Steinheim, Germany), VSL#3, VSL#3-derived *L. paracasei*, *L. plantarum* 299 v, *L. casei* BL23, *L. casei* ATCC 393 (type strain) or *E. coli* Nissle 1917 (24 h, moi 20, if not otherwise indicated).

**ELISA.** IP-10 (murine/human) and IL-6 (murine) concentrations in IEC supernatants were determined using the appropriate ELISA kits (R&D Europe, Abington, England) according to the manufacturers instructions. Serum amyloid A (SAA) concentrations in plasma were determined using the appropriate ELISA kit (Invitrogen, Karlsruhe, Germany) according to the manufacturers instructions.

### Example 2:

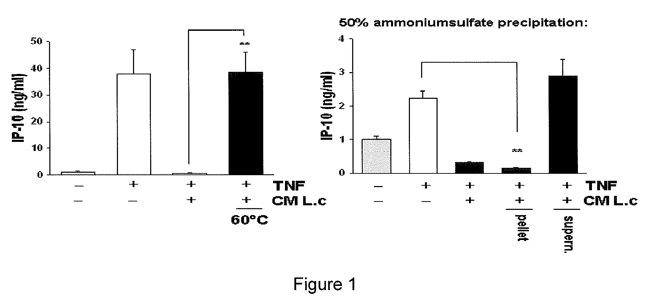

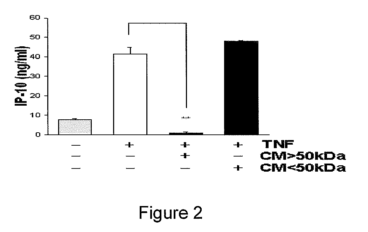

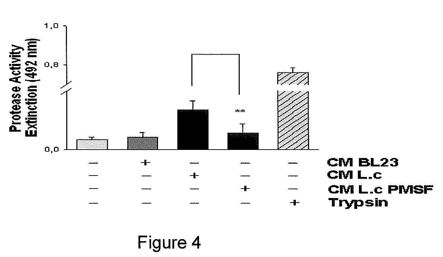

**The active bacterial component secreted by *L. paracasei* (VSL#3) is a bacterial protease.** Bacterial CM is a very complex suspension of abundant bacterial components like unmethylated DNA, metabolites, proteins and polysaccharides. In order to analyse, which specific bacterial component is responsible for the observed inhibition of IP-10 expression in IEC, CM was subjected to several treatments. Heat-treatment of CM *L. paracasei* (VSL#3) was found to result in inactivation of the active component (Figure 4) whereas Benzonase A treatment (data not shown) did not have any effect, excluding bacterial DNA as the active component. Activity was found to be precipitable by 50% ammonium sulfate (Figure 1), suggesting that the inhibition of IP-10 secretion is mediated via proteinaceous bacterial components.
Importantly, size exclusion experiments showed that the active protein is bigger than 50 kDa. The active component is concentrated in the retentate using a 50 kDa filter device, whereas the flow-through was found to be completely inactive (Figure 2).

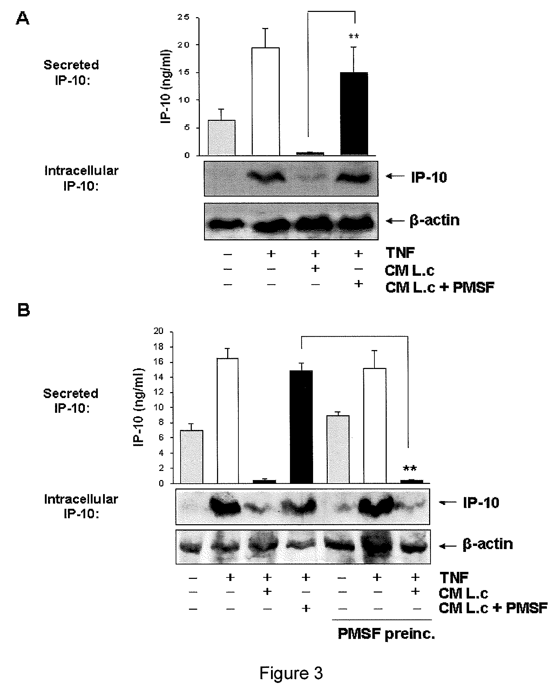

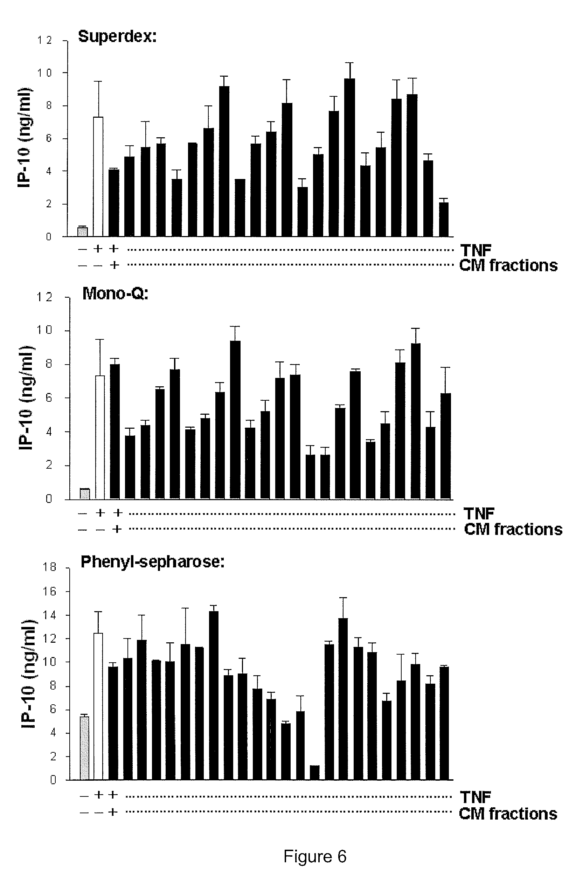

Surprisingly, PMSF, an irreversible serine/cysteine protease inhibitor, abrogated the inhibitory effect of CM *L. paracasei* (VSL#3) on IP-10 expression in IEC (Figure 6). This result raised the question of whether the PMSF-mediated effect was due to the inhibition of an active bacterial protease or due to the inhibition of a cellular protease involved in the degradation of IP-10 in IEC. Stimulation experiments with untreated CM *L*. *paracasei* (VSL#3) in PMSF-preincubated Mode K cells revealed that PMSF does not inhibit the degradation of IP-10 in IEC, but that it inhibits the activity of a bacterial protease in the CM of *L. paracasei* (VSL#3) (Figure 3).

Subsequent serine/cystein protease activity assays showed that CM *L. paracasei* (VSL#3) indeed contains one or more PMSF-sensitive serine/cysteine proteases in contrast to CM *L. casei* BL23. However, the proteolytic activity of CM *L. paracasei* (VSL#3) in the FTC-casein assay was found to be rather weak and was only observed when CM *L. paracasei* (VSL#3) was used in high concentrations (Figure 4). These results suggest that FTC-casein is not a preferred substrate for the active bacterial protease that mediates the loss of IP-10 in IEC.

### Example 3:

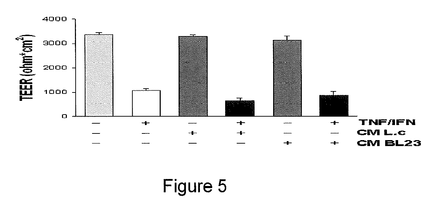

**The active bacterial protease does not reduce TEER.** Bacterial proteases were recently shown to be able to reduce intestinal barrier function (Steck et. al, 2009, Gastroenterology, 136-5, supplement 1, A21-A22). In order to investigate whether the secreted protease of *L. paracasei* (VSL#3) exerts similar detrimental effects on IEC barrier function, T84 cells were stimulated with CM *L. paracasei* (VSL#3) using CM *L. casei* BL23 as negative control. The active protease secreted by *L. paracasei* (VSL#3) did not reduce TEER (transepithelial electrical resistance) of T84 cells. In addition, CM *L*. *paracasei* (VSL#3) did not induce further decrease of TNF/IFNy-reduced TEER of T84 cells (Figure 5). This result shows that the active bacterial protease secreted by *L. paracasei* (VSL#3) does not negatively influence intestinal epithelial barrier function.

### Example 4:

**Lactocepin is the active bacterial protease of *L. paracasei* (VSL#3).** In order to characterize the active bacterial protease that is secreted by *L. paracasei* (VSL#3), chromatographic fractionation of CM *L. paracasei* (VSL#3) was performed. CM *L. paracasei* (VSL#3) was fractionated according to molecular mass (superdex chromatography), hydrophobicity (phenyl-sepharose) or charge (MonoQ-ion exchange chromatography). The resulting chromatographic fractions were subsequently screened in cell culture stimulation experiments with regard to their potential to reduce IP-10 secretion. Surprisingly, each of the three different chromatographic methods led to the elution of several active fractions, which were separated by non-active fractions (Figure 6). This uncommon elution profile of the active protein points towards an oligomeric protease with different active subunits, self-processing of the active protease during elution or the existence of several independently active proteases in the CM of *L. paracasei* (VSL#3).

Several chromatographic fractions that were identified to be highly active or non-active were subsequently subjected to LC-MS-MS analysis. All active fractions were found to contain a bacterial protease, lactocepin, that was absent from non-active fractions (Table 1). Lactocepin is a membrane-bound and secreted serine protease with a molecular weight of approximately 200 kDa (precursor), thereby fitting all the previously detected characteristics of the active component of *L. paracasei* (VSL#3). From this result it can be taken that lactocepin is the active probiotic structure that mediates the observed inhibition of IP-10 secretion in IEC.

**Table 1: Lactocepin was identified as the active protease secreted by L. paracasei (VSL#3).**

| | | | **chromatography fractions** | | | | | |
|---|---|---|---|---|---|---|---|---|
| **gene name** | **protein name** | **MW (kDa)** | **n.a** | **a** | **a** | **n.a** | **a** | **n.a** |
| *l* | D-2 hydroxyisocaproate dehydrogenase | 36 | + | + | | | | |
| groL | 60kDa chaperonin | 57 | + | + | + | | + | |
| valS | Valyl-tRNA synthethase- | 100 | + | + | | | | |
| gap | Glyceraldehyde-3-phosphate dehydrogenase | 36 | + | + | + | + | | + |
| queA | S-adenosylmethionine:tRNA ribosyltransferase | 38 | + | | | | | |
| gnd | 6-phosphogluconate dehydrogenase | 52 | + | + | | | | |
| groS | 10 kDa Chaperonin | 10 | | + | + | | | |
| **prtP** | **PII-type proteinase precursor (Lactocepin)** | 200 | | + | + | | + | |
| metG | MethionyltRMA synthetase | 76 | | | | + | | |
| dnaK | hsp70 | 66 | | | | + | | |
| Idh | L. lactatdehydrogenase | 35 | | | | | + | |
| galE | UDP-glucose 4-epimerase | 36 | | | | | + | |
| prtP | PI-type proteinase precursor | 199 | | | | | + | |
| purF | Amidophosphoribosyltransferase | 112 | | | | | + | |
| guaB | Inosine-5'-monophosphate dehydrogenase | 52 | | | | | + | |
| pgk | Phosphoglycerate kinase | 42 | | | | | | + |
| tuf | Elongation factor Tu | 43 | | | | | | + |

| | | | | | | | | |
|---|---|---|---|---|---|---|---|---|
| **n.a (not active)** **a (active)** | | | | | | | | |

Different active as well as non-active fractions derived from chromatographic separations were subjected to LC-MS-MS analysis. The table shows all proteins that were identified in the respective fractions. All active fractions were found to contain a cell wall associated and secreted serine protease, lactocepin, which was absent from the non-active fractions.

It has been shown that other lactobacillus strains like *L. casei* BL23 and *L. casei* ATCC334, the extracts of which were inactive with regard to the inhibition of IP-10 expression in IEC, in fact also encode, but do not produce lactocepins under the bacterial growth conditions applied in the present study. This means that under appropriate growth conditions said strains may also produce lactocepin, thus having the ability to degrade IP-10.

It has been found that under the following conditions specifically *L. paracasei* VSL#3 but not L. casei BL23 or *L*. *casei* ATCC334 secrete active lactocepin:
**Preparation of bacterial conditioned media (CM).** CM was generated by growing VSL#3-derived *L. paracasei, L. casei* BL23, *L. casei* ATCC393 and *L. plantarum* 299v (5x10⁷ cfu/ml) anaerobically in DMEM (1% Glutamine) containing HEPES (20 mM) (Invitrogen, Carlsbad, USA) (37°C, 5% CO₂, 24 h).

### Example 5:

The incubation of inflamed pieces of inflamed tissue with lactocepin containing CM of *L. paracasei* led to a significant reduction of IP-10 in the tissue supernatant, whereas the secretion of tumor necrosis factor was not influenced.

4x4 mm colonic tissue pieces of IL10-/- mice were incubated with or without lactocepin-containing bacterial supernatant from *L. paracasei* (VSL#3) in explant culture media for 24 h. After that period, the concentration of secreted IP-10 and TNF in the explant culture media was determined using ELISA analysis. It was found that the level of IP-10 was significantly reduced or even undetectable whereas the level of TNF secreted by the explants was unaffected by treatment with lactocepin-containing bacterial supernatant.

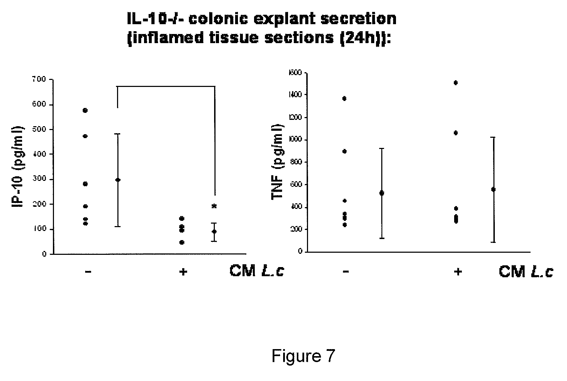

This result is shown in Figure 7.

### Example 6:

IP-10 as direct substrate of lactocepin of *L*. *paracasei.*

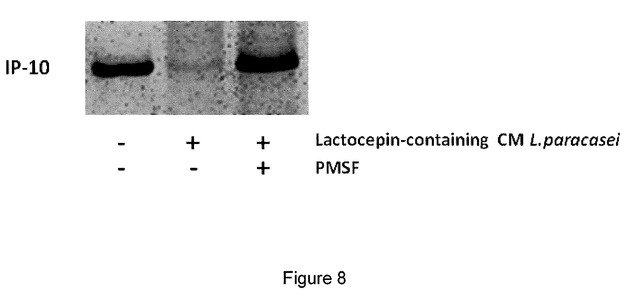

Recombinant IP-10 protein (250 ng) was incubated (o.n, 37°C) with lactocepin-containing conditioned media of *L. paracasei* (VSL#3) or lactocepin-containing conditioned media of *L. paracasei* (VSL#3) that was preincubated with PMSF (1 mM, 15 min). The mixture was then subjected to a SDS-PAGE separation and the recombinant IP-10 protein was made visible via Flamingo staining. Figure 8 shows that lactocepin completely degrades IP-10, whereas the degradation is inhibited by the irreversible inhibition of lactocepin via PMSF.

### Example 7:

IP-10 as direct substrate of lactocepin of *L. paracasei* vs. *L. casei* BL23.

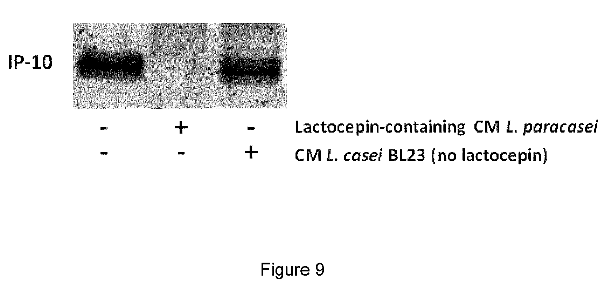

Recombinant IP-10 protein (250 ng) was incubated (o.n, 37°C) with lactocepin-containing conditioned media of *L. paracasei* (VSL#3) or conditioned media of *L. casei* BL23 (lacking lactocepin). The mixture was then subjected to a SDS-PAGE separation and the recombinant IP-10 protein was made visible via Flamingo staining. Figure 9 shows that only the lactocepin-containing CM of *L. paracasei* VSL#3 is able to degrade IP-10.

### Example 8:

Degradation of chemokines by the proteolytic action of lactocepin of *L*. *paracasei.*

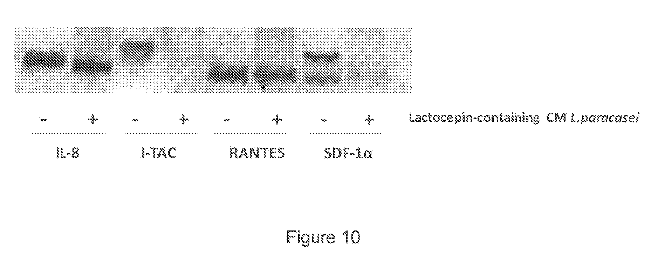

Various recombinant chemokines (250 ng) were incubated (o.n, 37°C) with lactocepin-containing conditioned media of *L. paracasei* (VSL#3). The mixtures were then subjected to a SDS-PAGE separation and the recombinant chemokines were made visible via Flamingo staining. Figure 10 shows that lactocepin is able to degrade I-TAC and SDF-1α whereas IL-8 and RANTES are not degraded.

### Example 9:

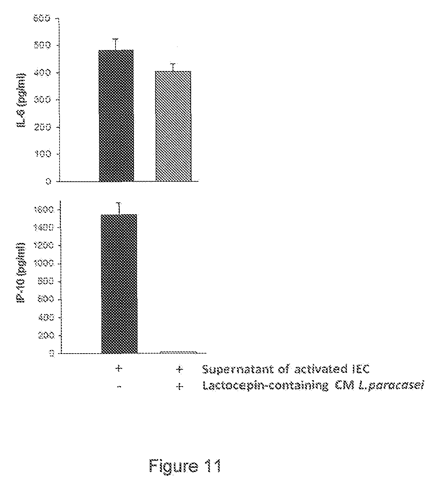

The IP-10 and IL-6 containing complex supernatant of TNF-activated IEC was incubated (o.n, 37°C) with lactocepin-containing conditioned media of *L. paracasei* (VSL#3) and the chemokine/cytokine levels were subsequently analysed via ELISA analysis. Figure 11 shows that lactocepin results in complete loss of IP-10 whereas IL-6 is unaffected by the bacterial protease.

### Example 10:

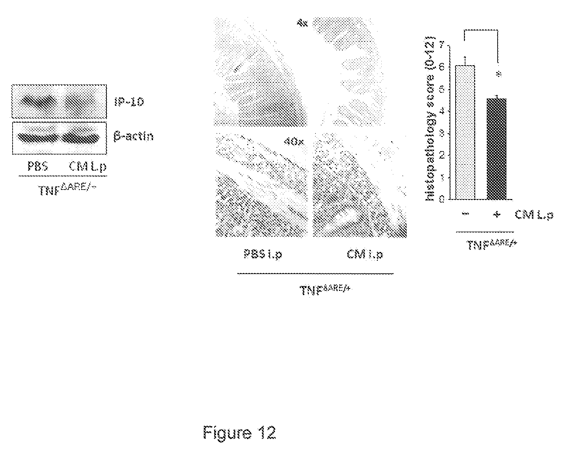

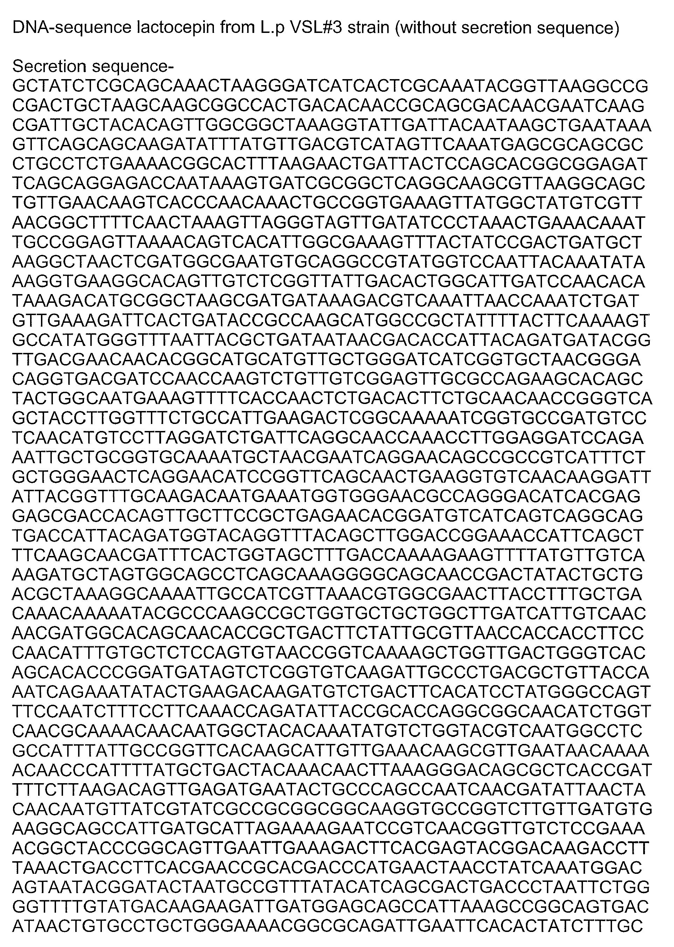

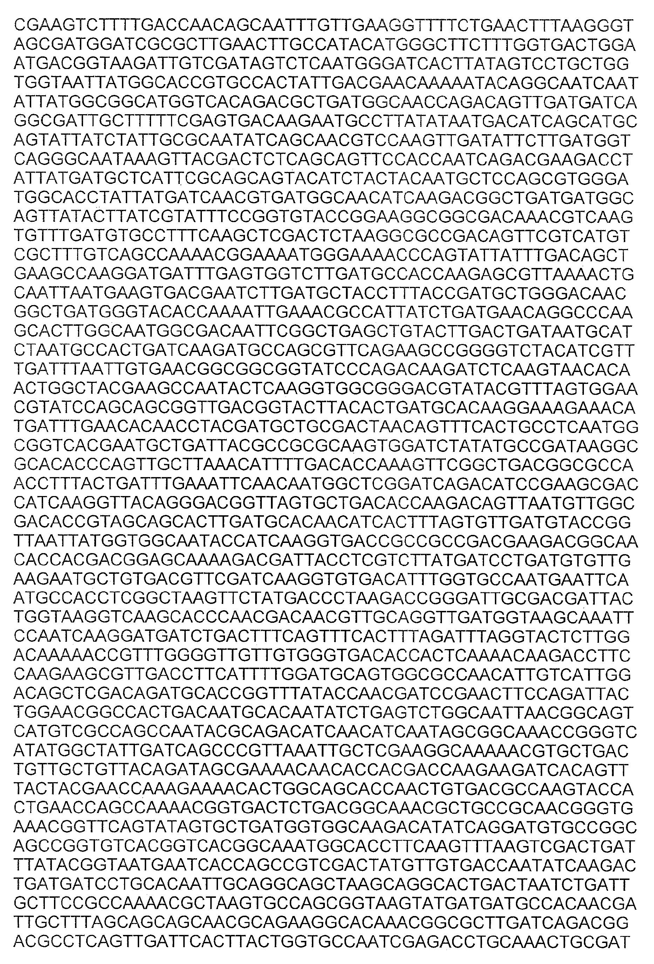

Three inflammated TNF^{ΔARE} mice were treated intraperitoneally with lactocepin-containing CM (in PBS). Every second day, 200 µl 150x concentrated CM were administered. Parallel thereto, three mice were treated with PBS alone. As can be seen from Fig. 12, a significant reduction of IP-10 in ileal tissue of CM-treated mice is achieved. Moreover, a significant reduction of the inflammation is observed.

## Claims

1. Lactocepins and/or a functionally active fragment thereof for use in the prophylaxis and/or the treatment of a disease.

2. One or more lactocepin(s) and/or one or more functionally active fragment(s) thereof for use in the prophylaxis and/or treatment of an inflammatory disease.

3. One or more lactocepin(s) and/or one or more functionally active fragment(s) thereof according to claim 2 for use in the prophylaxis and/or treatment of IP-10-mediated inflammatory diseases.

4. One or more lactocepin(s) and/or one or more functionally active fragment(s) thereof for use in the prophylaxis and/or treatment of an allergic reaction.

5. One or more lactocepin(s) and/or one or more functionally active fragment(s) thereof according to one of claims 2 to 4 for use in the prophylaxis and/or treatment of an Eotaxin-mediated allergic reaction.

6. The lactocepin(s) and/or functionally active fragment(s) thereof for use according to one of claims 1 to 5, wherein the lactocepin(s) and/or functionally active fragment(s) thereof are comprised in a pharmaceutical or a therapeutic nutritional composition.

7. The lactocepin(s) and/or functionally active fragment(s) thereof for use according to one of claims 1 to 6, wherein the composition further comprises one or more ingredients selected from physiologically compatible drugs, dietary supplements or pharmaceutically or physiologically acceptable excipients.

8. The lactocepin(s) and/or functionally active fragment(s) thereof for use according to one of claims 1 to 7, wherein the lactocepin is lactocepin derived from *L. paracasei* (VSL#3) or *Lactococcus lactis cremoris* SK11.

9. The lactocepin(s) and/or functionally active fragment(s) thereof for use according to one of claims 1 to 8, wherein the lactocepin is lactocepin derived from SEQ ID NO: 1.

10. The lactocepin(s) and/or functionally active fragment(s) thereof for use according to one of claims 3 to 9, wherein the IP-10-mediated inflammatory disease is selected from inflammatory bowel diseases, autoimmune diseases, chronic or acute inflammations, atherosclerosis, arthritis or coronary heart disease.

11. The lactocepin(s) and/or functionally active fragment(s) thereof for use according to one of claims 6 to 10, wherein the pharmaceutical composition is in a form for oral, parenteral, topical, in-joint or cutaneous administration.

## Patentansprüche

1. Lactocepine und/oder funktionell wirksame Fragmente davon zur Verwendung bei der Prophylaxe und/oder Behandlung einer Krankheit.

2. Ein oder mehrere Lactocepine und/oder ein oder mehrere funktionell wirksame Fragmente davon zur Verwendung bei der Prophylaxe und/oder Behandlung einer Entzündungskrankheit.

3. Ein oder mehrere Lactocepine und/oder ein oder mehrere funktionell wirksame Fragmente davon nach Anspruch 2 zur Verwendung bei der Prophylaxe und/oder Behandlung von IP-10-vermittelten Entzündungskrankheiten.

4. Ein oder mehrere Lactocepine und/oder ein oder mehrere funktionell wirksame Fragmente davon zur Verwendung bei der Prophylaxe und/oder Behandlung einer allergischen Reaktion.

5. Ein oder mehrere Lactocepine und/oder ein oder mehrere funktionell wirksame Fragmente davon nach einem der Ansprüche 2 bis 4 zur Verwendung bei der Prophylaxe und/oder Behandlung einer Eotaxinvermittelten allergischen Reaktion.

6. Lactocepin(e) und/oder funktionell wirksames Fragment/wirksame Fragmente davon zur Verwendung nach einem der Ansprüche 1 bis 5, worin das (die) Lactocepin(e) und/oder funktionell wirksame Fragment/wirksamen Fragmente davon in einer pharmazeutischen oder therapeutischen Ernährungszusammensetzung enthalten sind.

7. Lactocepin(e) und/oder funktionell wirksames Fragment/wirksame Fragmente davon zur Verwendung nach einem der Ansprüche 1 bis 6, worin die Zusammensetzung weiterhin einen oder mehrere Bestandteile umfasst, ausgewählt aus physiologisch kompatiblen Arzneimitteln, Nahrungsergänzungsmittel und pharmazeutisch oder physiologisch verträgliche Exzipienten.

8. Lactocepin(e) und/oder funktionell wirksames Fragment/wirksame Fragmente davon zur Verwendung nach einem der Ansprüche 1 bis 7, worin das Lactocepin Lactocepin ist, das von *L. paracasei* (VSL Nr.3) oder *Lactococcus lactis* cremoris SK11 stammt.

9. Lactocepin(e) und/oder funktionell wirksames Fragment/wirksame Fragmente davon zur Verwendung nach einem der Ansprüche 1 bis 8, worin das Lactocepin Lactocepin ist, das von SEQ ID NO:1 abgeleitet ist.

10. Lactocepin(e) und/oder funktionell wirksames Fragment/wirksame Fragmente davon zur Verwendung nach einem der Ansprüche 3 bis 9, worin die IP-10-vermittelte Entzündungskrankheit ausgewählt ist aus entzündlichen Darmerkrankungen, Autoimmunerkrankungen, chronischen oder akuten Entzündungen, Atherosklerose, Arthritis oder koronaren Herzkrankheiten.

11. Lactocepin(e) und/oder funktionell wirksames Fragment/wirksame Fragmente davon zur Verwendung nach einem der Ansprüche 6 bis 10, worin die pharmazeutische Zusammensetzung in einer Form zur oralen, parenteralen, topischen Verabreichung, Verabreichung ins Gelenk oder kutanen Verabreichung ist.

## Revendications

1. Lactocépines et/ou fragment fonctionnellement actif de celles-ci pour utilisation dans la prophylaxie et/ou le traitement d'une maladie.

2. Une ou plusieurs lactocépine(s) et/ou un ou plusieurs fragment(s) fonctionnellement actif(s) de celle(s)-ci pour utilisation dans la prophylaxie et/ou le traitement d'une maladie inflammatoire.

3. Une ou plusieurs lactocépine(s) et/ou un ou plusieurs fragment (s) fonctionnellement actif(s) de celle(s)-ci selon la revendication 2 pour utilisation dans la prophylaxie et/ou le traitement de maladies inflammatoires médiées par IP-10.

4. Une ou plusieurs lactocépine(s) et/ou un ou plusieurs fragment(s) fonctionnellement actif(s) de celle(s)-ci pour utilisation dans la prophylaxie et/ou le traitement d'une réaction allergique.

5. Une ou plusieurs lactocépine(s) et/ou un ou plusieurs fragment (s) fonctionnellement actif(s) de celle(s)-ci selon l'une quelconque des revendications 2 à 4, pour utilisation dans la prophylaxie et/ou le traitement d'une réaction allergique médiée par une éotaxine.

6. Lactocépine(s) et/ou fragment(s) fonctionnellement actif(s) de celle(s)-ci pour utilisation selon l'une quelconque des revendications 1 à 5, lesquels lactocépine(s) et/ou fragment(s) fonctionnellement actif(s) de celle(s)-ci sont compris dans une composition pharmaceutique ou nutritionnelle thérapeutique.

7. Lactocépine(s) et/ou fragment(s) fonctionnellement actif(s) de celle(s)-ci pour utilisation selon l'une quelconque des revendications 1 à 6, dans lesquels la composition comprend en outre un ou plusieurs ingrédient(s) choisi(s) parmi les substances médicamenteuses physiologiquement compatibles, les suppléments diététiques, ou les excipients pharmaceutiquement ou physiologiquement acceptables.

8. Lactocépine(s) et/ou fragment(s) fonctionnellement actif(s) de celle(s)-ci pour utilisation selon l'une quelconque des revendications 1 à 7, dans lesquels la lactocépine est une lactocépine issue de *L. paracasei* (VSL #3) ou *Lactococcus lactis cremoris* SK11.

9. Lactocépine(s) et/ou fragment(s) fonctionnellement actif(s) de celle(s)-ci pour utilisation selon l'une quelconque des revendications 1 à 8, dans lesquels la lactocépine est une lactocépine issue de SEQ ID NO: 1.

10. Lactocépine(s) et/ou fragment(s) fonctionnellement actif(s) de celle(s)-ci pour utilisation selon l'une quelconque des revendications 3 à 9, dans lesquels la maladie inflammatoire médiée par IP-10 est choisie parmi les maladies inflammatoires intestinales, les maladies auto-immunes, les inflammations chroniques ou aiguës, une athérosclérose, une arthrite ou une maladie cardiaque coronaire.

11. Lactocépine(s) et/ou fragment(s) fonctionnellement actif(s) de celle(s)-ci pour utilisation selon l'une quelconque des revendications 6 à 10, dans lesquels la composition pharmaceutique est sous une forme pour l'administration orale, parentérale, topique, intra-articulaire ou cutanée.


### SEQUENCE LISTING

<110> Technische Universitat München
<120> Lactocepins for use in the treatment of IP-10-mediated inflammatory diseases
<130> 49895P WO
<160> 1
<170> PatentIn version 3.3
<210> 1
   <211> 5514
   <212> DNA
   <213> Artificial
<220>
   <223> Sequence lactocepin from L.p VSL3 strain
<400> 1

gctatctcgc agcaaactaa gggatcatca ctcgcaaata cggttaaggc cgcgactgct 60

aagcaagcgg ccactgacac aaccgcagcg acaacgaatc aagcgattgc tacacagttg 120

gcggctaaag gtattgatta caataagctg aataaagttc agcagcaaga tatttatgtt 180

gacgtcatag ttcaaatgag cgcagcgcct gcctctgaaa acggcacttt aagaactgat 240

tactccagca cggcggagat tcagcaggag accaataaag tgatcgcggc tcaggcaagc 300

gttaaggcag ctgttgaaca agtcacccaa caaactgccg gtgaaagtta tggctatgtc 360

gttaacggct tttcaactaa agttagggta gttgatatcc ctaaactgaa acaaattgcc 420

ggagttaaaa cagtcacatt ggcgaaagtt tactatccga ctgatgctaa ggctaactcg 480

atggcgaatg tgcaggccgt atggtccaat tacaaatata aaggtgaagg cacagttgtc 540

tcggttattg acactggcat tgatccaaca cataaagaca tgcggctaag cgatgataaa 600

gacgtcaaat taaccaaatc tgatgttgaa agattcactg ataccgccaa gcatggccgc 660

tattttactt caaaagtgcc atatgggttt aattacgctg ataataacga caccattaca 720

gatgatacgg ttgacgaaca acacggcatg catgttgctg ggatcatcgg tgctaacggg 780

acaggtgacg atccaaccaa gtctgttgtc ggagttgcgc cagaagcaca gctactggca 840

atgaaagttt tcaccaactc tgacacttct gcaacaaccg ggtcagctac cttggtttct 900

gccattgaag actcggcaaa aatcggtgcc gatgtcctca acatgtcctt aggatctgat 960

tcaggcaacc aaaccttgga ggatccagaa attgctgcgg tgcaaaatgc taacgaatca 1020

ggaacagccg ccgtcatttc tgctgggaac tcaggaacat ccggttcagc aactgaaggt 1080

gtcaacaagg attattacgg tttgcaagac aatgaaatgg tgggaacgcc agggacatca 1140

cgaggagcga ccacagttgc ttccgctgag aacacggatg tcatcagtca ggcagtgacc 1200

attacagatg gtacaggttt acagcttgga ccggaaacca ttcagctttc aagcaacgat 1260

ttcactggta gctttgacca aaagaagttt tatgttgtca aagatgctag tggcagcctc 1320

agcaaagggg cagcaaccga ctatactgct gacgctaaag gcaaaattgc catcgttaaa 1380

cgtggcgaac ttacctttgc tgacaaacaa aaatacgccc aagccgctgg tgctgctggc 1440

ttgatcattg tcaacaacga tggcacagca acaccgctga cttctattgc gttaaccacc 1500

accttcccaa catttgtgct ctccagtgta accggtcaaa agctggttga ctgggtcaca 1560

gcacacccgg atgatagtct cggtgtcaag attgccctga cgctgttacc aaatcagaaa 1620

tatactgaag acaagatgtc tgacttcaca tcctatgggc cagtttccaa tctttccttc 1680

aaaccagata ttaccgcacc aggcggcaac atctggtcaa cgcaaaacaa caatggctac 1740

acaaatatgt ctggtacgtc aatggcctcg ccatttattg ccggttcaca agcattgttg 1800

aaacaagcgt tgaataacaa aaacaaccca ttttatgctg actacaaaca acttaaaggg 1860

acagcgctca ccgatttctt taagacagtt gagatgaata ctgcccagcc aatcaacgat 1920

attaactaca acaatgttat cgtatcgccg cggcggcaag gtgccggtct tgttgatgtg 1980

aaggcagcca ttgatgcatt agaaaagaat ccgtcaacgg ttgtctccga aaacggctac 2040

ccggcagttg aattgaaaga cttcacgagt acggacaaga cctttaaact gaccttcacg 2100

aaccgcacga cccatgaact aacctatcaa atggacagta atacggatac taatgccgtt 2160

tatacatcag cgactgaccc taattctggg gttttgtatg acaagaagat tgatggagca 2220

gccattaaag ccggcagtga cataactgtg cctgctggga aaacggcgca gattgaattc 2280

acactatctt tgccgaagtc ttttgaccaa cagcaatttg ttgaaggttt tctgaacttt 2340

aagggtagcg atggatcgcg cttgaacttg ccatacatgg gcttctttgg tgactggaat 2400

gacggtaaga ttgtcgatag tctcaatggg atcacttata gtcctgctgg tggtaattat 2460

ggcaccgtgc cactattgac gaacaaaaat acaggcaatc aatattatgg cggcatggtc 2520

acagacgctg atggcaacca gacagttgat gatcaggcga ttgctttttc gagtgacaag 2580

aatgccttat ataatgacat cagcatgcag tattatctat tgcgcaatat cagcaacgtc 2640

caagttgata ttcttgatgg tcagggcaat aaagttacga ctctcagcag ttccaccaat 2700

cagacgaaga cctattatga tgctcattcg cagcagtaca tctactacaa tgctccagcg 2760

tgggatggca cctattatga tcaacgtgat ggcaacatca agacggctga tgatggcagt 2820

tatacttatc gtatttccgg tgtaccggaa ggcggcgaca aacgtcaagt gtttgatgtg 2880

cctttcaagc tcgactctaa ggcgccgaca gttcgtcatg tcgctttgtc agccaaaacg 2940

gaaaatggga aaacccagta ttatttgaca gctgaagcca aggatgattt gagtggtctt 3000

gatgccacca agagcgttaa aactgcaatt aatgaagtga cgaatcttga tgctaccttt 3060

accgatgctg ggacaacggc tgatgggtac accaaaattg aaacgccatt atctgatgaa 3120

caggcccaag cacttggcaa tggcgacaat tcggctgagc tgtacttgac tgataatgca 3180

tctaatgcca ctgatcaaga tgccagcgtt cagaagccgg ggtctacatc gtttgattta 3240

attgtgaacg gcggcggtat cccagacaag atctcaagta acacaactgg ctacgaagcc 3300

aatactcaag gtggcgggac gtatacgttt agtggaacgt atccagcagc ggttgacggt 3360

acttacactg atgcacaagg aaagaaacat gatttgaaca caacctacga tgctgcgact 3420

aacagtttca ctgcctcaat ggcggtcacg aatgctgatt acgccgcgca agtggatcta 3480

tatgccgata aggcgcacac ccagttgctt aaacattttg acaccaaagt tcggctgacg 3540

gcgccaacct ttactgattt gaaattcaac aatggctcgg atcagacatc cgaagcgacc 3600

atcaaggtta cagggacggt tagtgctgac accaagacag ttaatgttgg cgacaccgta 3660

gcagcacttg atgcacaaca tcactttagt gttgatgtac cggttaatta tggtggcaat 3720

accatcaagg tgaccgccgc cgacgaagac ggcaacacca cgacggagca aaagacgatt 3780

acctcgtctt atgatcctga tgtgttgaag aatgctgtga cgttcgatca aggtgtgaca 3840

tttggtgcca atgaattcaa tgccacctcg gctaagttct atgaccctaa gaccgggatt 3900

gcgacgatta ctggtaaggt caagcaccca acgacaacgt tgcaggttga tggtaagcaa 3960

attccaatca aggatgatct gactttcagt ttcactttag atttaggtac tcttggacaa 4020

aaaccgtttg gggttgttgt gggtgacacc actcaaaaca agaccttcca agaagcgttg 4080

accttcattt tggatgcagt ggcgccaaca ttgtcattgg acagctcgac agatgcaccg 4140

gtttatacca acgatccgaa cttccagatt actggaacgg ccactgacaa tgcacaatat 4200

ctgagtctgg caattaacgg cagtcatgtc gccagccaat acgcagacat caacatcaat 4260

agcggcaaac cgggtcatat ggctattgat cagcccgtta aattgctcga aggcaaaaac 4320

gtgctgactg ttgctgttac agatagcgaa aacaacacca cgaccaagaa gatcacagtt 4380

tactacgaac caaagaaaac actggcagca ccaactgtga cgccaagtac cactgaacca 4440

gccaaaacgg tgactctgac ggcaaacgct gccgcaacgg gtgaaacggt tcagtatagt 4500

gctgatggtg gcaagacata tcaggatgtg ccggcagccg gtgtcacggt cacggcaaat 4560

ggcaccttca agtttaagtc gactgattta tacggtaatg aatcaccagc cgtcgactat 4620

gttgtgacca atatcaagac tgatgatcct gcacaattgc aggcagctaa gcaggcactg 4680

actaatctga ttgcttccgc caaaacgcta agtgccagcg gtaagtatga tgatgccaca 4740

acgattgctt tagcagcagc aacgcagaag gcacaaacgg cgcttgatca gacggacgcc 4800

tcagttgatt cacttactgg tgccaatcga gacctgcaaa ctgcgatcaa tcaattagct 4860

gccaaattac ctgcagataa gaagacttcg ctgcttaacc agttgcaatc tgtgaaggct 4920

gcgctgggaa cggacttggg caatcaaact gatccaagca ctggcaaaac atttacggca 4980

gcgttagacg agctagtggc acaagctcaa gcaggcacgc aaacggccga ccagcttcaa 5040

gcgagtcttg ccaaggtact tgatgcagta ttagcaaaac ttgcggaggg tattaaagcg 5100

gcaacaccgg ctgaggttgg caatgctaaa gatgctgcaa ctggcaaaac ttggtatgcc 5160

gacattgctg acacattgac gtctggtcaa gccagtgctg atgcgtctga caagcttgca 5220

catttacaag ctttgcaaag tctgaaaacg aaggtggcag ctgccgttga agcggccaag 5280

acagctggta aaggcgacga tacagccggt actagcgaca aaggcggcgg tcaaggtacc 5340

ccggcgcccg ctccaggcga cacaggtaag gacaaaggcg atgagggcag ccagcctagt 5400

tctggcggta atatcccaac aaagccagcc acaacgacgt caacgagcac ggatgatacg 5460

actgatcgta atggtcaaca tacatccggt aagggagcat tacccaagac ataa 5514